# EUROPEAN PATENT APPLICATION

(11) **EP 4 173 954 A1**
(43) Date of publication of application: **03.05.2023**
(21) Application number: 21205977.8
(22) Date of filing: 02.11.2021
(51) Int. Cl.: B64D 13/00

(54) **AIRCRAFT FRAGRANCE DEVICE**

(71) Applicant: B/E Aerospace, Inc., Winston Salem, NC 27105 (US)
(72) Inventor: CASTILLO GONZALEZ, Arnau, 3439 MG Nieuwegein (NL)
(74) Representative: Dehns

(57) **Abstract**

A method of fragrance release within an aircraft (20) having a cabin (40) and an environmental control system (60) for controlling the cabin environment, the method comprising: receiving a current aircraft status from the aircraft's environmental control system (60), wherein the current aircraft status comprises one of: boarding, take-off, breakfast service, lunch service, dinner service, beverage service, sleep mode, cruise, landing, deboarding and emergency; determining an appropriate fragrance for the current aircraft status from a plurality of fragrance sources; and releasing the appropriate fragrance for the current aircraft status into the aircraft's cabin (40).

## Description

The present disclosure relates to a method of fragrance release within an aircraft and an aircraft fragrance device.

Aircraft provide a unique environment for passengers compared to other forms of transport. Typically passengers are seated within a cabin that is maintained at a pressure lower than that of ambient pressure at sea level. The lower pressure causes, amongst other things, changes in passengers sense of taste. For example, salt may be perceived as much as 20-30% less intense and sugar may be perceived as much as 15-20% less intense. In response to the reduced perceived intensity of certain ingredients, such as salt and sugar, meals provided to aircraft passengers are typically provided with higher quantities of these ingredients. This means that meals provided on aircraft may have quantities of ingredient, such as salt and sugar, significantly above the recommended levels by health authorities. The alternative is that food is provided that tastes poor due to the change in taste perception caused by the lower pressure.

Further the nature of aircraft, for example enclosed spaces, limited visibility and certain flight phases such as take-off, landing and turbulence are known to cause passengers stress and discomfort.

A need therefore exists for a means of improving passengers' experience aboard aircraft.

Viewed from a first aspect, a method of fragrance release within an aircraft having a cabin and an environmental control system for controlling the cabin environment is provided, the method comprising: receiving a current aircraft status from the aircraft's environmental control system, wherein the current aircraft status comprises one of: boarding, take-off, breakfast service, lunch service, dinner service, beverage service, sleep mode, cruise, landing, deboarding and emergency; determining an appropriate fragrance for the current aircraft status from a plurality of fragrance sources; and releasing the appropriate fragrance for the current aircraft status into the aircraft's cabin.

Viewed from a second aspect, an aircraft fragrance device for an aircraft having a cabin and an environmental control system for controlling the cabin environment is provided, comprising: a fragrance source holder configured to retain a plurality of fragrance sources; and a fragrance controller configured to receive an aircraft status from the environmental control system and activate an appropriate fragrance source from the plurality of fragrance sources for the aircraft's current aircraft status in order to release the appropriate fragrance from the appropriate fragrance source into the cabin, wherein the current aircraft status comprises one of: boarding, take-off, breakfast service, lunch service, dinner service, beverage service, sleep mode, cruise, landing, deboarding and emergency.

According to a further aspect, there is provided an aircraft comprising: an aircraft fragrance device as described above; and an environmental control system, wherein the environmental control system is configured to communicate the aircraft status to the aircraft fragrance device.

Further optional features of the above aspects are set out by the dependent claims.

Certain preferred embodiments of the present disclosure will now be described in greater detail, by way of example only and with reference to the following figures, in which:
Figure 1 shows an aircraft fragrance device on an aircraft;
Figure 2 shows a schematic illustration of the components of the aircraft fragrance device; and
Figure 3 shows a fragrance device fragrance release sequence.

Viewed from a first aspect, a method of fragrance release within an aircraft having a cabin and an environmental control system for controlling the cabin environment is provided, the method comprising: receiving a current aircraft status from the aircraft's environmental control system, wherein the current aircraft status comprises one of: boarding, take-off, breakfast service, lunch service, dinner service, beverage service, sleep mode, cruise, landing, deboarding and emergency; determining an appropriate fragrance for the current aircraft status from a plurality of fragrance sources; and releasing the appropriate fragrance for the current aircraft status into the aircraft's cabin.

In this example the aircraft's cabin environment, specifically the scents within the cabin, are varied automatically in response to the current aircraft status. This advantageously provides a pleasant experience for those people on board the aircraft whilst minimising any burden on the crew to monitor, determine, select and release the appropriate fragrance for the current aircraft status. A fragrance, as discussed herein, should be understood to include a combination or blend of fragrances, and so a single fragrance source may for example include multiple fragrances combined to achieve multiple effects. In addition, as discussed below, fragrances from multiple fragrance sources may be released at the same time to achieve different effects.

The appropriate fragrance may be selected to induce a synaesthetic effect for people within the cabin. In other words the appropriate fragrance may be a fragrance that causes other senses of those people within the cabin to be enhanced, i.e. senses other than smell. One example is that a fragrance may be released that enhances taste perception. In the alternative, or in addition, the fragrance may be selected for a synaesthetic effect known to impact on the mood or emotion of a person.

When the aircraft status corresponds to one of: breakfast service, lunch service, dinner service or beverage service, the appropriate fragrance may be a fragrance that induces a synaesthetic effect that enhances the perception of taste. The appropriate fragrance may be a fragrance that induces a synaesthetic effect that enhances the perception of at least one of salt or sugar. An advantage of this effect is that quantities of ingredients in food or beverages that may be above health guidance can be reduced without negatively affecting the passengers taste perception. This therefore improves the healthiness of the food and beverages that can be provided without negatively impacting the passenger experience.

When the aircraft status corresponds to one of take-off and landing the appropriate fragrance may be a fragrance that induces a synaesthetic effect that reduces stress.

When the aircraft status corresponds to one of boarding or deboarding the appropriate fragrance may be a fragrance that corresponds to a signature fragrance of the aircraft's operator. In this case the fragrance may enhance the first impression and may allow people to identify the airline via the fragrance. This may be done in addition to a synaesthetic effect on mood, emotion or senses.

When the aircraft status corresponds to a cruise status the appropriate fragrance may be a fragrance that enhances comfort and awareness of in-flight services.

When the aircraft status corresponds to a sleep status the appropriate fragrance may be a fragrance that facilitates sleep. This therefore benefits the passengers restfulness and supports their general well-being.

When the aircraft status corresponds to an emergency status the appropriate fragrance may be a fragrance that enhances a state calmness and increases awareness, for example it may be a fragrance known to enhance hearing perception.

The method may comprise detecting each of the plurality of fragrance sources via at least one identifier corresponding to each fragrance source. The at least one identifier may be one of a bar code, QR code, NFC or RFID corresponding to each fragrance source.

Releasing the appropriate fragrance may comprise evaporating fragrance from an appropriate fragrance source into the aircraft's cabin air supply. This advantageously allows for all areas of the cabin to receive a substantially uniform fragrance intensity. In other words, the fragrance intensity is not limited to the locality of a fragrance release device.

The appropriate fragrance may be evaporated by activating a piezoelectric device for the appropriate fragrance source.

The appropriate fragrance may be a combination of two or more of the plurality of fragrance sources. This advantageously allows for a greater number of fragrances to be released for a set of fragrance sources. In other words, fewer fragrance sources are required to release the appropriate fragrance.

Two or more fragrances may be evaporated at predetermined rates to release the appropriate fragrance.

The method may comprise using an aircraft fragrance device as discussed below, or optional features thereof.

Viewed from a second aspect, an aircraft fragrance device for an aircraft having a cabin and an environmental control system for controlling the cabin environment is provided, comprising: a fragrance source holder configured to retain a plurality of fragrance sources; and a fragrance controller configured to receive an aircraft status from the environmental control system and activate an appropriate fragrance source from the plurality of fragrance sources for the aircraft's current aircraft status in order to release the appropriate fragrance from the appropriate fragrance source into the cabin, wherein the current aircraft status comprises one of: boarding, take-off, breakfast service, lunch service, dinner service, beverage service, sleep mode, cruise, landing, deboarding and emergency.

In this example a device is provided that advantageously allows scents within the cabin, to be varied automatically in response to the current aircraft status. The device hence contributes to providing a pleasant experience for those people on board the aircraft whilst minimising any burden on the crew to monitor, determine, select and release the appropriate fragrance for the current aircraft status.

The aircraft fragrance device may be configured to be installed in-line with an aircraft's cabin air supply. This advantageously allows for all areas of the cabin to receive a substantially uniform fragrance intensity. In other words, the fragrance intensity is not limited to the locality of the fragrance release device.

The aircraft fragrance device may be configured to be retrofitted to existing an aircraft's cabin air supply. In other words, the aircraft fragrance device may have a form that can be inserted into existing aircraft cabin air supplies. This hence increases the compatibility of the aircraft fragrance device whilst also reducing the installation and manufacturing costs.

The aircraft fragrance device may comprise a detector configured to detect at least one identifier present on each fragrance source. The at least one identifier may be at least one of a bar code, QR code, NFC or RFID.

The aircraft fragrance device may comprise a piezoelectric evaporator configured to cause evaporation of the appropriate fragrance from the fragrance sources responsive to a command from the controller.

According to a further aspect, there is provided an aircraft comprising: an aircraft fragrance device as described above; and an environmental control system, wherein the environmental control system is configured to communicate the aircraft status to the aircraft fragrance device.

The aircraft may comprise: a lighting system; and a sound system; wherein the environmental control system is configured to adapt the lighting and sound dependent on the aircraft status.

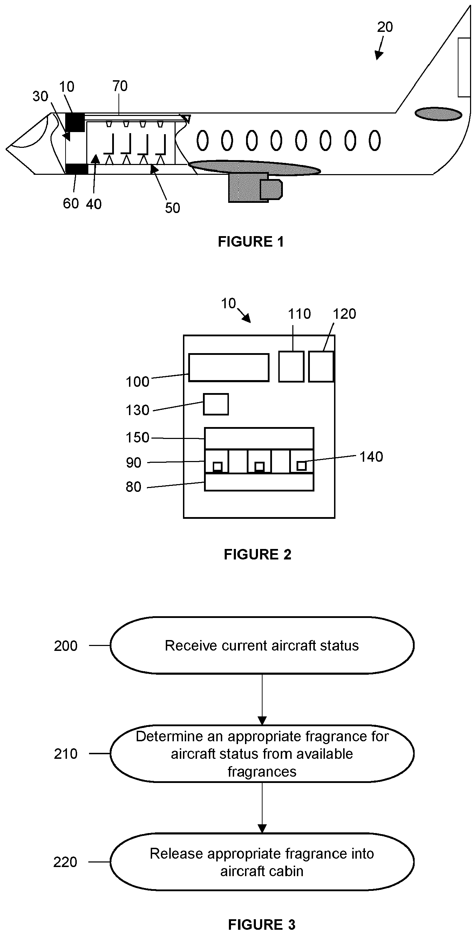

Figure 1 shows an aircraft fragrance device 10 installed on an aircraft 20 within an overhead portion of a galley 30. The aircraft 20 has a cabin 40, which in this example contains a plurality of seats 50 to seat passengers. The aircraft 20 also has an environmental control system 60.

The aircraft fragrance device 10 is configured to receive from the environmental control system 60 data indicating the current aircraft status. The aircraft status may be one boarding, take-off, breakfast service, lunch service, dinner service, beverage service, sleep mode, cruise, landing, deboarding and emergency. Based on the current aircraft status the aircraft fragrance device 10 is configured to activate and release an appropriate fragrance into the cabin 40.

In the illustrated example, activating the appropriate fragrance consists of evaporating via a piezoelectric evaporator of the aircraft fragrance device 10 the appropriate fragrance. The evaporated fragrance is then distributed via the aircraft's cabin air supply 70 into the cabin 40.

Figure 2 shows in more detail the components of the aircraft fragrance device 10. The aircraft fragrance device 10 comprises a fragrance source holder 80; a plurality of fragrance sources 90; a fragrance controller 100, an air inlet 110; an air outlet 120; a detector 130 and a piezoelectric electric evaporator 150.

The fragrance source holder 80 is configured to hold a plurality of fragrance sources 90 and is further configured to allow the fragrance sources 90 to be removed and inserted into the fragrance device 10. For example, when a fragrance source 90 is depleted or out of date it may be removed and a replacement may be reinserted and held by the fragrance source holder 80. The fragrance sources 90 may comprise an identifier 140 such as a bar code, QR code, NFC or RFID that can be detected by the detector 130.

The fragrance controller 100 is configured to receive an aircraft status from the environmental control system as well as information regarding what fragrance sources 90 are available via the detector 130 reading the identifier 140 on each fragrance source 90. Based on the aircraft status the fragrance controller 100 activates the piezoelectric evaporator 150 to evaporate the appropriate fragrance for the current aircraft status.

The aircraft fragrance device 10 has an air inlet 110 and an air outlet 120 such that it can be installed in-line with aircraft's cabin air supply 70. Therefore, when the fragrance controller 100 activates the piezoelectric evaporator 150 the evaporated fragrance is entrained in the air flow flowing through the aircraft's cabin air supply 70 and is released into the cabin 40.

Figure 3 shows a fragrance device fragrance release sequence. At step 200 the aircraft fragrance device 10 receives the current aircraft status. The current aircraft status may be one of: boarding, take-off, breakfast service, lunch service, dinner service, beverage service, sleep mode, cruise, landing, deboarding and emergency.

At step 210 the controller 100 of the aircraft fragrance device 10 determines the appropriate fragrance for the aircraft status from the available fragrances 90. The appropriate fragrance being a fragrance that induces a desired synaesthetic effect. For example when the aircraft status corresponds to dinner service, or more specifically dessert service, the appropriate fragrance may be vanilla, which is known to cause a synaesthetic effect of increasing the perception of sweetness.

At step 220 the piezoelectric evaporator 150 is activated to evaporate the appropriate fragrance from a fragrance source 90 such that it entrained in the cabin air supply 70 and dispersed into the cabin 40.

## Claims

1. A method of fragrance release within an aircraft having a cabin and an environmental control system for controlling the cabin environment, the method comprising:
receiving a current aircraft status from the aircraft's environmental control system, wherein the current aircraft status comprises at least one of: boarding, take-off, breakfast service, lunch service, dinner service, beverage service, sleep mode, cruise, landing, deboarding and emergency;
determining an appropriate fragrance for the current aircraft status from a plurality of fragrance sources; and
releasing the appropriate fragrance for the current aircraft status into the aircraft's cabin.

2. The method of claim 1, wherein the appropriate fragrance is selected to induce a synaesthetic effect for people within the cabin.

3. The method of claim 2, wherein when the aircraft status corresponds to one of:
breakfast service, lunch service, dinner service or beverage service, the appropriate fragrance is a fragrance that induces a synaesthetic effect that enhances the perception of taste.

4. The method of claim 2, wherein when the aircraft status corresponds to one of take-off and landing the appropriate fragrance is a fragrance that induces a synaesthetic effect that reduces stress.

5. The method of any preceding claim, comprising detecting each of the plurality of fragrance sources via at least one identifier corresponding to each fragrance source.

6. The method of any preceding claim, wherein releasing the appropriate fragrance comprises evaporating fragrance from an appropriate fragrance source into the aircraft's cabin air supply.

7. The method of any preceding claim, wherein the appropriate fragrance is evaporated by activating a piezoelectric device for the appropriate fragrance source.

8. The method of any preceding claim, wherein the appropriate fragrance is a combination of two or more of the plurality of fragrance sources.

9. The method of claim 8, wherein two or more fragrances are evaporated at predetermined rates to release the appropriate fragrance.

10. An aircraft fragrance device for an aircraft having a cabin and an environmental control system for controlling the cabin environment, comprising:
a fragrance source holder configured to retain a plurality of fragrance sources; and
a fragrance controller configured to receive an aircraft status from the environmental control system and activate an appropriate fragrance source from the plurality of fragrance sources for the aircraft's current aircraft status in order to release the appropriate fragrance from the appropriate fragrance source into the cabin, wherein the current aircraft status comprises at least one of: boarding, take-off, breakfast service, lunch service, dinner service, beverage service, sleep mode, cruise, landing, deboarding and emergency.

11. The aircraft fragrance device of claim 10, wherein the aircraft fragrance device is configured to be installed in-line with an aircraft's cabin air supply.

12. The aircraft fragrance device of claim 10 or 11, comprising a detector configured to detect at least one identifier on each fragrance source.

13. The aircraft fragrance device of any of claims 10 to 12, comprising a piezoelectric evaporator configured to cause evaporation of the appropriate fragrance from the fragrance sources responsive to a command from the controller.

14. An aircraft, comprising:
the aircraft fragrance device of any of claims 10 to 13; and
an environmental control system,
wherein the environmental control system is configured to communicate the aircraft status to the aircraft fragrance device.

15. The aircraft of claim 14, comprising:
a lighting system; and
a sound system;
wherein the environmental control system is configured to adapt the lighting and sound dependent on the aircraft status.
